# FASCICULE DE BREVET EUROPEEN

(11) **EP 1 372 550 B1**
(45) Date de publication et mention de la délivrance du brevet: **08.03.2006**
(21) Numéro de dépôt: 02722352.8
(22) Date de dépôt: 20.03.2002
(51) Int. Cl.: A61F 5/01

(54) **ORTHESE TRAPEZO-METACARPIENNE**
MITTELHAND-FINGERGELENK ORTHESE
TRAPEZO-METACARPAL ORTHOSIS

(30) Priorité: 26.03.2001 FR 0104133
(43) Date de publication de la demande: 02.01.2004
(73) Titulaire: Marc, Thierry, 34070 Montpellier (FR)
(72) Inventeur: Marc, Thierry, 34070 Montpellier (FR)
(74) Mandataire: Gosse, Michel
(86) Numéro de dépôt international: PCT/FR2002/000968
(87) Numéro de publication internationale: WO 2002/080826

(56) Documents cités:
- DE-A- 3 519 493
- DE-A- 3 631 253
- FR-A- 2 650 175
- FR-A- 2 662 075
- US-A- 1 471 948
- US-A- 4 709 694
- US-A- 5 356 371
- US-A- 6 101 628

## Description

L'invention concerne une orthèse pouce-poignet pour le traitement de pathologies relatives à l'articulation trapézo-métacarpienne et sa région.

La base du pouce est souvent le siège de douleurs qui peuvent parfois être invalidantes et gêner la fonction de la main.
Ces douleurs surviennent dans le cadre postraumatique (entorse trapézo-métacarpienne) ou dans une pathologie dégénérative (rhizarthrose, tendinopathie de De Quervain).
Le but du traitement est de mettre la colonne du pouce au repos.
Cette mise au repos est obtenue en plaçant le pouce en antéposition. Dans cette position la première commissure est ouverte et la base du premier métacarpien et bien centrée sur le trapèze, les structures capsuloligamentaires et tendino-musculaires sont détendues.
Cette position permet une cicatrisation des tissus en position correcte après traumatisme.
Dans les atteintes rhumatologiques les douleurs diminuent et l'on peut espérer ralentir la vitesse de dégénérescence.
Dans la rhizarthrose, l'évolution se fait vers une fermeture de la première commissure, d'une part, et la luxation externe de la base du premier métacarpien, d'autre part.
Les orthèses doivent donc s'opposer à cette tendance.

Les orthèses existantes sont de deux types.
1) Les orthèses statiques
   Elles sont en général en matériau moulé sur le patient (métal, matériau thermoformable). Leur but est d'ouvrir la première commissure soit en y moulant une "C-Barre" soit en tirant le pouce dans le sens de l'ouverture avec un appui sur la base de la première phalange.
   Ces orthèses sont, soit moulées directement sur le patient, soit de série mais avec une plaque qui devra être moulée sur le patient par un homme de l'art puis glissée dans le fourreau fabriqué en série.
   Ces orthèses statiques permettent un bon positionnement mais gênent considérablement l'utilisation de la main dans les activités de la vie quotidienne.
2) Les orthèses dynamiques
   Leur conception fait appel en général à des matériaux élastiques qui doivent permettre une mobilité de la colonne du pouce.
   Leur principe est d'exercer une traction vers l'extérieur sur l'articulation métacarpo-phalangienne du pouce pour ouvrir la première commissure.
   Certaines, comme l'orthèse du pouce de Thuasne, exercent en plus une action de recentrage sur la base du premier métacarpien.
   Leur principal défaut est de présenter des problèmes de tolérance à cause de la faible surface de l'appui sur l'articulation méta-phalagienne qui exerce à lui seul la force d'ouverture.
   Le brevet US5356371 décrit une orthèse pourvue d'une éclisse rigide que l'on déplace pour accroître la mobilité du pouce et un moyen de maintien de ladite orthèse au poignet.
   Le brevet US1471948 décrit une orthèse qui comporte une éclisse élastique autorisant une certaine mobilité au pouce.

L'orthèse, selon l'invention, met en oeuvre trois fondions thérapeutiques agissant en combinaison.
- La première consiste à placer, dans la première commissure, un matériau élastique (déformation et retour élastique) épousant la forme de la commissure (comme les "C-Barres" statiques). Ce matériau déformable et souple permet une utilisation quasi-normale de la main lors de la préhension (ouverture de la commissure pour la préparation à la prise puis fermeture de celle-ci lors de la prise). Lorsque la main revient au repos, le pouce est repositionné automatiquement (de par l'élasticité du matériau) en antéposition.
- La deuxième consiste à placer au niveau de l'articulation trapézo-métacarpienne (base du pouce), un dispositif de serrage du poignet qui a pour effet d'exercer une force de recentrage de la base du premier métacarpien. Cette force (intensité) peut être réglée indépendamment de l'ouverture de la commissure.
- La troisième consiste à mettre en place un fourreau destiné à recevoir des éclisses (élastiques ou rigides) qui vont permettre de rigidifier et de fixer la correction amenée par l'orthèse (première phase après un traumatisme) ou, si le matériau est une lame ressort élastique, d'augmenter la force d'ouverture de la commissure sans perdre l'intérêt fonctionnel de l'orthèse.

Le fourreau est placé sur la face dorsale du pouce sur le trajet du long extenseur pour augmenter l'effet d'écartement.
Une fonction additionnelle consiste à réaliser les orthèses en élastomère du type "Néoprène" de manière à augmenter la chaleur autour du pouce. Cette chaleur a un effet antalgique bien connu et apprécié dans les cas de rhumatismes.

Les caractéristiques et les avantages de l'invention vont apparaître plus clairement à la lecture de la description détaillée qui suit d'au moins un mode de réalisation préféré de celle-ci donné à titre d'exemple non-limitatif et représenté aux dessins annexés.
Sur ces dessins :

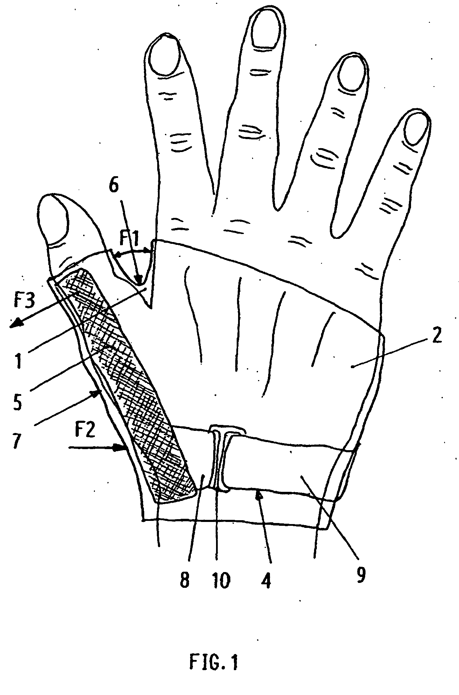

- la figure 1 est une vue dorsale de la main équipée de l'orthèse selon l'invention;

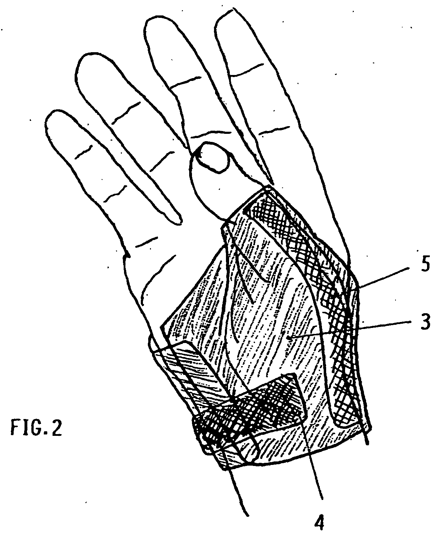

- la figure 2 est une vue palmaire de la main équipée de ladite orthèse;

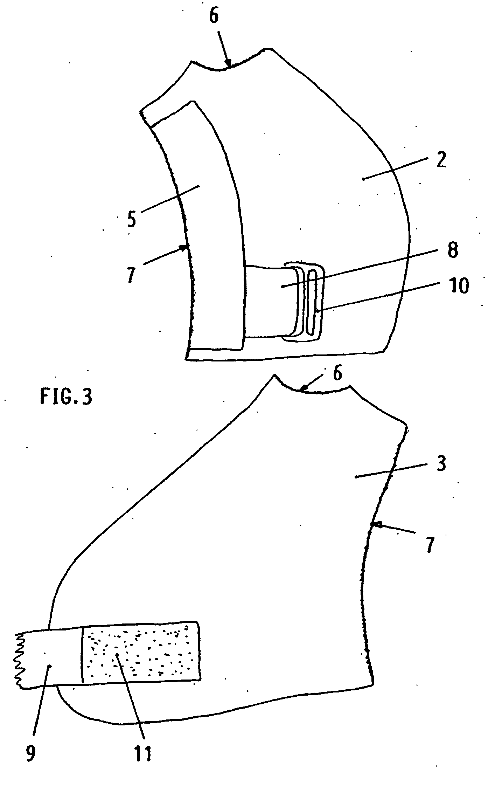

- la figure 3 est une vue à plat des deux pièces constitutives de l'orthèse avant assemblage.

L'orthèse pouce-poignet pour le traitement de pathologies relatives à l'articulation trapézo-métacarpienne et à sa région, représentée aux figures, comporte trois moyens ayant des effets thérapeutiques combinés :
- le premier moyen, placé entre le pouce et l'index, dans la première commissure, réalisé en un matériau (1), épousant la forme de ladite commissure, appartenant, pour partie, à la face dorsale (2) de l'orthèse et, pour autre partie, à sa face palmaire (3), est adapté pour permettre, d'une part, de par sa souplesse, la fermeture de ladite commissure à chaque sollicitation et, d'autre part, de par son élasticité, l'ouverture automatique (F1) de ladite commissure lorsque l'action de fermeture précédente cesse ainsi que le maintien du pouce, au repos, en antéposition;
- le deuxième moyen, placé au niveau de l'articulation trapézo-métacarpienne, autrement dit à la base du pouce, servant de dispositif de serrage (4), réglable en intensité, est adapté pour exercer une force (F2) de recentrage de la base du premier métacarpien;
- le troisième moyen, placé sur la face dorsale du pouce, sur le trajet du long extenseur, du type fourreau (5) destiné à recevoir une éclisse, réalisée en matériau élastique, est adapté pour augmenter l'effet d'écartement (F3) du matériau (1) placé dans la première commissure.

Selon la réalisation préférée de l'invention :
- l'orthèse est réalisée en deux parties, une dorsale (2) et une palmaire (3), reliées au moyen de deux coutures, la première (6) située dans le plan médian de la première commissure et la deuxième (7) située sur la face latérale externe (radiale) du pouce ; la première couture ayant pour effet de renforcer l'élasticité du premier moyen ;
- le dispositif de serrage du deuxième moyen est une sangle réalisée en deux parties (8) et (9) reliées, par l'une de leurs extrémités, respectivement aux pièces dorsale (2) et palmaire (3) et, par l'autre de leurs extrémités, à un moyen d'attache réglable du type boucle (10) et moyen auto-agrippant (11), notamment du type "Velcro";
- les pièces dorsale (2) et palmaire (3) sont réalisées en un élastomère du type "Néoprène".
   L'éclisse élastique peut être remplacée temporairement par une éclisse rigide pendant la première phase du traitement après un traumatisme.

Bien entendu, l'invention n'est pas limitée aux modes de réalisation décrits et représentés pour lesquels on pourra prévoir d'autres variantes, en particulier dans :
- la nature et la forme des sous-ensembles constitutifs de l'orthèse;
- la conception des moyens combinant les effets F1, F2 et F3.

## Revendications

1. Orthèse pouce-poignet pour le traitement des pathologies relatives à l'articulation trapézo-métacarpienne et sa région, **caractérisée en ce qu'**elle comporte, en combinaison, trois moyens (1,4,5) ayant des effets thérapeutiques combinés :
a) le premier moyen (1), épousant la forme de la première commissure et réalisé en un matériau élastique, est adapté pour permettre, d'une part, de par sa souplesse, la fermeture de ladite commissure à chaque sollicitation et, d'autre part, de par son élasticité, l'ouverture automatique (F1) de celle-ci, lorsque l'action de fermeture précédente cesse, afin de maintenir le pouce, au repos, en antéposition ;
b) le deuxième moyen (4), placé au niveau de l'articulation trapézo-métacarpienne, autrement dit à la base du pouce, est adapté pour exercer une force (F2) de recentrage de la base du premier métacarpien ;
c) le troisième moyen (5), placé sur la face dorsale du pouce, sur le trajet du long extenseur, du type fourreau destiné à recevoir une éclisse réalisée en matériau élastique, est adapté pour augmenter la force d'ouverture (F3) du premier moyen (1) placé dans la première commissure.

2. Orthèse, selon la revendication 1, **caractérisée en ce qu'**elle est réalisée en deux parties, une dorsale (2) et une palmaire (3), reliées au moyen de deux coutures, la première (6) située dans le plan médian de la première commissure et la deuxième (7) située sur la face latérale externe (radiale) du pouce.

3. Orthèse, selon la revendication 1, **caractérisée en ce que** le dispositif de serrage du deuxième moyen est une sangle réalisée en deux parties (8) et (9) reliées, par l'une de leurs extrémités, respectivement aux pièces dorsale (2) et palmaire (3) et, par l'autre de leurs extrémités, à un moyen d'attache réglable du type boucle (10) et moyen auto-agrippant (11).

## Patentansprüche

1. Daumen-Handgelenk-Orthese zur Behandlung von Krankheiten des Trapeziometakarpalgelenks und dessen Bereichs, **dadurch gekennzeichnet, dass** sie kombiniert drei Hilfsmittel (1,4,5) mit kombinierter therapeutischer Wirkung aufweist:
a) wobei das erste Hilfsmittel (1), das der Form der ersten Kommissur folgt und aus einem elastischen Material besteht, so beschaffen ist, dass es aufgrund seiner Biegsamkeit bei jeder Beanspruchung das Schließen der Kommissur und andererseits aufgrund seiner Dehnbarkeit deren automatisches Öffnen (F1) gestattet, sobald der vorhergehende Schließvorgang zu Ende ist, um so den Daumen im Ruhezustand in der vorderen Stellung zu halten;
b) wobei das zweite Hilfsmittel (4), das in Höhe des Trapeziometakarpalgelenks positioniert wird, also an der Basis des Daumens, so beschaffen ist, dass es eine Kraft (F2) zur erneuten Zentrierung der Basis des ersten Mittelhandknochens ausübt;
c) wobei das dritte Hilfsmittel (5), das auf der dorsalen Daumenseite auf der Bahn des langen Streckmuskels positioniert wird und eine Art Hülle darstellt, die dazu bestimmt ist, eine aus elastischem Material bestehende Schiene aufzunehmen, so beschaffen ist, dass es die Öffnungskraft (F3) des in der ersten Kommissur angeordneten ersten Hilfsmittels (1) erhöht.

2. Orthese nach Anspruch 1, **dadurch gekennzeichnet, dass** sie aus zwei Teilen gefertigt ist, und zwar einem dorsalen (2) und einem palmaren (3), die mittels zweier Schnitte miteinander verbunden sind, wobei sich der erste (6) auf der Mittelebene der ersten Kommissur und der zweite (7) auf der äußeren (radialen) Seitenfläche des Daumens befinden.

3. Orthese nach Anspruch 1, **dadurch gekennzeichnet, dass** es sich bei der Spannvorrichtung des zweiten Hilfsmittels um einen Gurt aus zwei Teilen (8) und (9) handelt, die an einem ihrer Enden mit dem dorsalen (2) bzw. dem palmaren (3) Stück und mit dem anderen Ende mit einem regulierbaren Befestigungsmittel vom Typ Schnalle (10) und einem selbstspannenden Mittel (11) verbunden sind.

## Claims

1. Thumb-wrist orthosis for the treatment of pathologies relative to the trapezius-metacarpus joint and its area, **characterised in that** it comprises, combined, three means (1,4,5) having combined therapeutic effects:
a) the first means (1) taking the exact shape of the first line of junction and made of an elastic material, is adapted to allow, on the one hand, because of its flexibility, to close the said line of junction at each stress and, on the other hand, because of its flexibility, the automatic opening (F1) thereof, when the preceding closing action stops, in order to keep the thumb, at rest, in anteposition;
b) the second means (4), placed at the level of the trapeziusmetacarpus joint, in other words, at the bottom of the thumb, is adapte for exerting a re-centring force (F2) of the bottom of the first metacarpus;
c) the third means (5), placed on the thumb back face, on the path of the sleeve-like long extensor, designed to receive a splint made of elastic material, is adapted to increase the opening force (F3) of the first means (1) located at the first line of junction.

2. Orthosis, according to claim 1, **characterised in that** it is made of two parts, a dorsal (2) and a palmar (3) parts, joined by two seams, the first seam (6) located within the median plane of the first line of junction and the second seam (7) located on the (radial) external side face of the thumb.

3. Orthosis, according to claim 1, **characterised in that** the holding device of the second means is a strap made of two parts (8) and (9) linked, by one of their ends, respectively to the dorsal (2) and palmar (3) parts, and by the other end, to a buckle-like adjustable fastening means (10) and selfgripping means (11).
